# FASCICULE DE BREVET EUROPEEN

(11) **EP 0 799 420 B1**
(45) Date de publication et mention de la délivrance du brevet: **30.05.2001**
(21) Numéro de dépôt: 95943256.8
(22) Date de dépôt: 21.12.1995
(51) Int. Cl.: G01N 33/53, G01N 33/543, C12Q 1/68, C07H 21/00

(54) **PROCEDE DE DOSAGE D'UN HAPTENE SELON UNE TECHNIQUE DE COMPETITION**
VERFAHREN ZUR KOMPETITIVEN BESTIMMUNG EINES HAPTENS
HAPTENE ASSAY BY A COMPETITION-BASED METHOD

(30) Priorité: 21.12.1994 FR 9415418
(43) Date de publication de la demande: 08.10.1997
(73) Titulaire: BIO MERIEUX, 69280 Marcy l'Etoile (FR)
(72) Inventeur: CROS, Philippe, F-69005 Lyon (FR); BATTAIL, Nicole, F-69002 Lyon (FR); PIGA, Nadia, F-69130 Ecully (FR)
(74) Mandataire: Tonnellier, Jean-Claude
(86) Numéro de dépôt international: FR9501709
(87) Numéro de publication internationale: WO9619729

(56) Documents cités:
- EP-A- 0 620 439
- WO-A-91/19812
- WO-A-93/20074
- WO-A-93/20094
- US-A- 4 921 788

### LISTE DE SEQUENCES

### (1) INFORMATIONS GENERALES

(i) DEPOSANT
   (A) NOM : BIO MERIEUX S.A.
   (B) RUE : Chemin de l'Orme
   (C) VILLE : Marcy l'Étoile
   (E) PAYS : France
   (F) CODE POSTAL : 69280
   (G) TELEPHONE : (33) 78 87 20 00
   (H) TELECOPIE : (33) 78 87 20 90
(ii) TITRE DE L'INVENTION : Procédé de dosage d'un haptène selon une technique de compétition.
(iii) NOMBRE DE SEQUENCES : 3
(iv) FORME LISIBLE PAR ORDINATEUR
   (A) TYPE DE SUPPORT : Floppy disk
   (B) ORDINATEUR : IBM PC Compatible
   (C) SYSTEME D'EXPLOITATION : MS-DOS 6.21
   (D) LOGICIEL : WORD 6.0 pour WINDOWS

### INFORMATIONS POUR LA SEQ ID NO: 1

(i) CARACTERISTIQUES DE LA SEQUENCE:
   (A) LONGUEUR: 22 nucléotides
   (B) TYPE: acide nucléique
   (C) NOMBRE DE BRINS: simple
   (D) CONFIGURATION: linéaire
(ii) TYPE DE MOLECULE: ADN
(iii) HYPOTHETIQUE: NON
(iv) ANTI SENS: NON
(vi) ORIGINE:
   (A) ORGANISME : oligonucléotide de synthèse 57
(ix) CARACTERISTIQUE:
   (D) CARACTERISTIQUE ADDITIONNELLE: Bras alkylamine (AMINOLINK2) à l'extrémité 5'
(xi) DESCRIPTION DE LA SEQUENCE: SEQ ID NO: 1 :

ACTAAAAACT AGTAATGCAA AG.......................................................22

### INFORMATIONS POUR LA SEQ ID NO: 2

(i) CARACTERISTIQUES DE LA SEQUENCE:
   (A) LONGUEUR: 21 nucléotides
   (B) TYPE: acide nucléique
   (C) NOMBRE DE BRINS: simple
   (D) CONFIGURATION: linéaire
(ii) TYPE DE MOLECULE: ADN
(iii) HYPOTHETIQUE: NON
(iv) ANTI SENS: NON
(vi) ORIGINE:
   (A) ORGANISME : oligonucléotide de synthèse 196
(ix) CARACTERISTIQUE:
   (D) CARACTERISTIQUE ADDITIONNELLE: nucléosides d'anomérie α et bras alkylamine (AMINOLINK2) à l'extrémité 5'
(xi) DESCRIPTION DE LA SEQUENCE: SEQ ID NO: 2 :

ACCCCGAGAT TTACGTTATG T.......................................................21

### INFORMATIONS POUR LA SEQ ID NO: 3

(i) CARACTERISTIQUES DE LA SEQUENCE:
   (A) LONGUEUR: 22 nucléotides
   (B) TYPE: acide nucléique
   (C) NOMBRE DE BRINS: simple
   (D) CONFIGURATION: linéaire
(ii) TYPE DE MOLECULE: ADN
(iii) HYPOTHETIQUE: NON
(iv) ANTI SENS: NON
(vi) ORIGINE:
   (A) ORGANISME : oligonucléotide de synthèse 2466
(ix) CARACTERISTIQUE:
   (D) CARACTERISTIQUE ADDITIONNELLE: Bras alkylamine (AMINOLINK2) à l'extrémité 5'
(xi) DESCRIPTION DE LA SEQUENCE: SEQ ID NO: 3 :

CTTTGCATTA CTAGTTTTTA GT ........................................................................22

## Description

La présente invention a pour objet un réactif pour le dosage d'haptènes, et son utilisation.

Les haptènes sont des molécules non immunogènes, c'est-à-dire incapables par elles mêmes de promouvoir une réaction immunitaire par production d'anticorps, mais capables d'être reconnues par des anticorps obtenus par immunisation d'animaux dans des conditions connues, en particulier par immunisation avec un conjugué haptène-protéine.

Diverses techniques ont été proposées pour le dosage d'haptènes dans un échantillon, parmi lesquelles les techniques dites de compétition. Ces techniques, décrites pour la première fois par Yalow et Berson (Nature, 184, p. 1648, 1959) pour le dosage de l'insuline plasmatique par radio-immunologie, ont été appliquées au dosage des haptènes au début des années 1970 (Gharig H. et al. J. Clin. Endocrinol. Metab., 31, p.709-714, 1970; Abraham G.E., Acta Endocrinologica, suppl. 183, p.1-42 1974).

Selon ces techniques, l'haptène que l'on cherche à doser est mis en compétition, pour sa fixation à une quantité définie d'anticorps spécifiques dudit haptène, avec une quantité connue, ajoutée, du même haptène marqué. Après une étape de séparation dans laquelle on sépare l'haptène libre (c'est-à-dire non lié aux anticorps) et les complexes anticorps-haptène, la quantité d'haptène marqué, lié ou libre, est déterminée grâce à l'activité du marqueur. Cette mesure permet de déduire la quantité d'haptène non marqué présente au départ. Dans ce type de dosage en compétition, la quantité de complexe haptène marqué-anticorps formé diminue au fur et à mesure qu'augmente la quantité d'haptène non marqué dans le milieu réactionnel.

Selon un premier mode de réalisation de ce type de dosage en compétition, l'haptène à doser et l'haptène marqué réagissent simultanément avec une quantité prédéterminée d'anticorps spécifiques de l'haptène. Après équilibre, les haptènes libres sont séparés des haptènes fixés aux anticorps. Selon un autre mode de réalisation, les réactifs sont ajoutés de manière séquentielle : dans un premier temps, l'haptène à doser est ajouté à une quantité d'anticorps prédéterminée de telle sorte que tout l'haptène à doser soit fixé. Dans un deuxième temps, l'haptène marqué est ajouté en excès afin de saturer les sites anticorps restés libres, et après un temps d'incubation suffisant, on sépare l'haptène marqué non lié.

Différents modes de marquage des haptènes ont été proposés. Le marquage radio-actif, bien qu'il offre l'avantage d'être suffisamment sensible, présente différents inconvénients tels que la labilité du marqueur et la nécessité d'un équipement lourd, de mesures de protection du personnel et d'élimination des déchets, qui constituent des contraintes importantes, notamment en ce qui concerne l'automatisation du test de dosage d'haptènes. Une autre possibilité consiste à utiliser comme marqueur une enzyme telle que par exemple la peroxydase de raifort, la phosphatase alcaline ou la β-galactosidase. L'addition du substrat spécifique de l'enzyme permet de révéler la quantité d'haptène marqué libre ou fixé aux anticorps (Van Weemen B.K. et Schuurs A.H.W.M., FEBS Letters, 24(1), p.77-81, 1972 ou Wisdom G.B., Clin. Chem., 22(8), p.1243-1255, 1976). Ce type de marquage a de nombreux avantages, tels que le coût, la simplicité d'utilisation, la rapidité de la réaction enzymatique, l'absence de mesures de protection particulières et la possibilité d'automatiser le test.

Il est toutefois connu des spécialistes que les méthodes de compétition utilisant un haptène marqué par une enzyme sont parfois peu sensibles et sont susceptibles de donner dans certains cas des résultats peu significatifs.

Le brevet US 4 921 788 décrit un procédé de dosage d'un haptène utilisant une technique de compétition pour la fixation sur des anticorps anti-haptène. Le compétiteur est un conjugué haptène-oligonucléotide, et la détection est mise en oeuvre avec une sonde oligonucléotidique qui ne peut former un duplex avec l'oligonucléotide du conjugué que si ce dernier n'est pas lié aux anticorps anti-haptène. La détection du conjugué non lié aux anticorps est effectuée à l'aide de bromure d'éthidium, qui présente une affinité pour les duplex nucléiques.

Le document EP-A-0620 439 décrit des anticorps capables de reconnaître des acides nucléiques.

La présente invention a pour objet un réactif pour le dosage d'haptènes par une technique de compétition qui permet de pallier les inconvénients précités.

Plus précisément, l'invention a pour objet un procédé pour le dosage d'un haptène dans un échantillon, dans lequel : (a) on met en contact ledit échantillon avec une quantité prédéterminée d'anticorps capables de reconnaître ledit haptène et avec une quantité prédéterminée d'un réactif capable d'entrer en compétition avec ledit haptène pour la fixation sur lesdits anticorps par formation d'un complexe de type antigène-anticorps, ledit réactif étant un conjugué formé par un fragment d'acide nucléique simple brin avec un composé capable d'être reconnu par les anticorps reconnaissant l'haptène, et (b) on détermine, à l'aide d'un marqueur capable de se lier avec ledit réactif, la quantité dudit réactif fixé sur lesdits anticorps ou la quantité dudit réactif non fixé sur lesdits anticorps, ledit marqueur étant un anticorps capable de reconnaître ledit fragment d'acide nucléique. On en déduit alors la quantité d'haptène présent dans l'échantillon.

Dans des modes de réalisation particuliers, on détermine la quantité de réactif fixé ou non fixé en mesurant la quantité de marqueur lié audit réactif ; pour cela, on ajoute le marqueur capable de se lier avec ledit réactif et l'on détermine la quantité de réactif fixé ou non fixé en mesurant la quantité de marqueur lié soit au réactif fixé sur les anticorps anti-haptène, soit au réactif libre, non fixé ; ledit marqueur est un anticorps capable de reconnaître ledit fragment d'acide nucléique du conjugué ; cet anticorps peut être lui-même lié, de façon connue, à un agent traceur, en vue de la détection ; cet anticorps peut également être détecté, de façon connue, par un anti-anticorps lui-même lié à un agent traceur.

Le procédé de l'invention peut être mis en oeuvre par mise en contact simultanée de l'échantillon et du réactif avec les anticorps capables de reconnaître l'haptène ; par exemple, dans ce cas, on peut mélanger l'échantillon et le réactif avant la mise en contact avec les anticorps.

Le procédé de l'invention peut également être mis en oeuvre de façon séquentielle. Dans ce cas, on peut dans un premier temps mettre en contact l'échantillon avec les anticorps capables de reconnaître l'haptène et dans un second temps ajouter le réactif.

Dans la suite de la description, on désignera par le terme "composé" le constituant du conjugué qui est lié au fragment d'acide nucléique. Ledit constituant est un composé reconnu par les anticorps reconnaissant l'haptène à doser. Ce composé peut être soit l'haptène lui-même, soit un analogue de cet haptène.

Dans la présente demande, le terme "haptène" désigne des composés ayant généralement une masse moléculaire inférieure à 3000 Da, et le plus souvent inférieure à 2000 Da, qui peuvent être par exemple des peptides, des peptides glycosylés, des métabolites, des vitamines, des hormones, des prostaglandines, des toxines ou divers médicaments.

L'haptène peut être choisi par exemple parmi :
- un peptide glycosylé tel que la séquence N-terminale de la sous-unité bêta de l'hémoglobine humaine ;
- les hormones thyroïdiennes, notamment la thyroxine, la triiodothyronine et la tétraiodothyronine; les hormones stéroïdiennes, notamment les estrogènes tels que l'estriol et l'estradiol, les androgènes tels que la testostérone, les progestatifs tels que la progestérone, les glucocorticoïdes tels que la 11-désoxycorticostérone et le cortisol ; les catécholamines telles que l'adrénaline, la noradrénaline, la dopamine ;
- les vitamines A, B comme la B12, C, D, E et K, l'acide folique, la biotine, la thiamine ;
- des médicaments tels que la digoxine, la digitoxigénine, la digitoxine, la digoxigénine; les antibiotiques notamment les aminoglycosides, la gentamicine, la tobramycine, l'amikacine, la sisomicine, la kanamycine, la netilmicine, la pénicilline, la tétracycline, la tétramycine, la chloromycétine et l'actinomycétine; ou le phénobarbital, la primidone, l'éthosuximide, la carbamazépine, le valproate, la théophylline, la caféine, le propanolol, le procainamide, la quinine, l'amitriptyline, la désipramine, le disopyramide, les amphétamines, la morphine, la méthadone, les barbituriques ;
- les nucléosides et nucléotides comme l'adénosine di- ou triphosphate (ADP et ATP), la flavine mononucléotide (FMN), la nicotinamide adénine dinucléotide (NAD), son dérivé phosphate (NADP), la thymidine, la guanosine et l'adénosine
- les toxines comme l'alphatoxine, la toxine du choléra, l'entérotoxine B du staphylocoque et analogues.

Dans la présente demande, le terme "anticorps" inclut les anticorps polyclonaux ou monoclonaux, les anticorps obtenus par recombination génétique, et des fragments d'anticorps tels que des fragments Fab ou F(ab')₂.

Les anticorps capables de reconnaître l'haptène sont également appelés ici "anticorps anti-haptène".

Le fragment d'acide nucléique simple brin présent dans le conjugué est notamment choisi dans le groupe consistant en un fragment d'ADN, un fragment d'ARN, un fragment hybride ADN/ARN ou un fragment d'ADN ou d'ARN modifié. On peut utiliser en particulier un fragment simple brin non susceptible d'auto-appariement.

Généralement, le fragment d'acide nucléique simple brin ne contient pas plus de 100 nucléotides, et en particulier pas plus de 40 nucléotides.

Le fragment d'acide nucléique est par exemple un oligodésoxyribonucléotide ou un oligoribonucléotide comprenant de 2 à 100 nucléotides, et plus particulièrement de 10 à 40 nucléotides, ou un désoxyribonucléotide ou un ribonucléotide, ou encore un désoxyribonucléoside ou un ribonucléoside.

Le fragment d'acide nucléique peut être un fragment d'acide nucléique modifié présentant par exemple une ou plusieurs bases modifiées ou peut être composé uniquement de bases modifiées naturelles (telles que la 6-céto-purine, la xanthine, la 5-méthyl-cytosine, la 2-amino-purine) ou non naturelles (telles que la thioguanine ou la 8-oxo-guanine). Le fragment d'acide nucléique peut être composé uniquement ou partiellement de nucléosides modifiés sur la partie osidique, par exemple des carbonucléosides, ou peut présenter des modifications au niveau du squelette phosphodiester, par exemple des groupements phosphorothioates. De même il peut être composé totalement ou partiellement de nucléosides d'anométrie alpha ou bêta ou d'isomères de la série D ou L. Un fragment d'acide nucléique modifié contient au moins une modification telle que celles qui viennent d'être mentionnées.

Le réactif utilisé dans le procédé de la présente demande est un conjugué résultant de la liaison d'un fragment d'acide nucléique avec un composé qui est soit l'haptène à doser soit un analogue de cet haptène, ledit analogue étant capable d'être reconnu par des anticorps reconnaissant l'haptène à doser.

Plus précisément, le fragment d'acide nucléique et le composé constitué par l'haptène ou par ledit analogue d'haptène sont liés par l'intermédiaire d'une liaison covalente pour former une molécule composite appelée "conjugué". Pour cela, on peut utiliser les méthodes connues d'établissement d'une liaison covalente. Par exemple, lorsque 'le fragment d'acide nucléique est préparé par synthèse automatique, l'haptène peut être additionné comme un nucléotide au cours de la synthèse, en position 5' et/ou 3' ou en toute autre position dans la chaîne nucléotidique. Cela nécessite préalablement la préparation d'un dérivé de l'haptène utilisable en synthèse automatique, par exemple, un dérivé phosphoramidite, H-phosphonate ou phosphotriester, ou la préparation d'un nucléotide substitué par ledit haptène sur la base purique ou pyrimidique, ou sur la partie osidique. Ledit haptène peut aussi être introduit au cours de la synthèse sur les phosphores internucléotidiques par phosphoramidation oxydative. Sur ces méthodes, on peut citer par exemple la revue faite par S.L. Beaucage et R. P. Iver, Tetrahedron 49, 1925-1963 (1993).

On peut également préparer des dérivés du fragment d'acide nucléique et de l'haptène de façon à introduire sur chacun une fonction réactive, les fonctions introduites étant capables de réagir l'une sur l'autre, avec formation d'une liaison covalente. A titre d'exemple, une fonction amine primaire portée par l'un des dérivés réagira avec l'ester de N-hydroxysuccinimide de l'autre dérivé qui contient un groupe carboxylique, de même une fonction thiol réagira sur un groupement maléimide, pyridyldisulfure ou halogénoalkyle et un phosphorothioate réagira avec un halogénoalkyle.

On peut aussi introduire sur le fragment d'acide nucléique et sur ledit composé (haptène ou analogue d'haptène) des fonctions réactives permettant une liaison par l'intermédiaire d'un agent de couplage.

Le terme "agent de couplage" tel qu'utilisé ici désigne une molécule contenant au moins deux groupements réactifs de même nature ou de nature différente (agent de couplage homobifonctionnel ou hétérobifonctionnel). A titre d'exemple, une fonction amine primaire et une fonction acide carboxylique pourront former une liaison amide en présence d'un agent de couplage tel qu'un carbodiimide, ou bien deux fonctions amines primaires peuvent être couplées en présence d'un agent de couplage tel que le phénylène-1,4-diisothiocyanate ou le disuccinimidylsubérate.

Les fonctions réactives introduites (ou déjà présentes) sur le fragment d'acide nucléique peuvent être choisies, par exemple, parmi les groupes amine, hydrazine, hydrazone, imine, imide, amide éventuellement substitué, semicarbazone, carbonyle (notamment un groupe aldéhyde), sulfure, thiol, carbamate, nitrile, halogène, hydroxyle, sulfonamide, isocyanate, isothiocyanate, acide carboxylique, ester d'acide carboxylique, halogénure d'acide carboxylique, anhydride d'acide, époxyde, phosphate, phosphorothioate, phosphite, phosphonate, thiophosphate, etc. Les fonctions réactives peuvent être temporairement protégées notamment lors de la synthèse des oligonucléotides.

Les fonctions réactives qui peuvent être introduites (ou éventuellement présentes) sur l'autre constituant du conjugué (c'est-à-dire sur composé qui est soit l'haptène soit un analogue de l'haptène), en vue de la réaction avec le dérivé d'acide nucléique, peuvent être choisies, par exemple, parmi les mêmes groupes que ceux qui viennent d'être énumérés, étant entendu que pour former le conjugué on choisit un dérivé d'haptène portant une fonction réactive capable de réagir, selon le cas, soit avec la fonction réactive présente sur le dérivé du fragment d'acide nucléique, soit avec une fonction réactive présente sur l'agent de couplage.

L'introduction de ces fonctions réactives sur les fragments d'acide nucléique, et par conséquent le greffage dudit composé sur le fragment nucléotidique, peut s'effectuer en toute position de la chaîne nucléotidique en utilisant par exemple des réactifs synthétisés selon des méthodes connues ou disponibles dans le commerce. A titre d'exemple les modifications peuvent être réalisées en position 5' par emploi d'un réactif du type Aminolink II (Applied Biosystems réf. 400808), ou en position 3' par utilisation d'un support solide tel que celui vendu par la société Clontech Lab. Inc sous la référence 5221 ou décrit par Reed et al. (1991) Bioconjugate Chem. 2,217-225. Ces modifications peut être effectuées sur les bases nucléiques (Glen Research réf. 101039), ou bien sur la partie osidique par modification de la partie osidique en utilisant par exemple un ribofuranose substitué en position 2' à la place d'un désoxy-2'-ribofuranose (Yamana et al. (1991) Tetrahedron Lett. 32, 6347-6350), ou en substituant un nucléoside par un résidu alkylamine (Clontech Lab. Inc réf. 5203) ou encore par substitution d'un phosphate internucléotidique selon des protocoles décrits par Froehler et al., (1988) Nucl. Acids Res., 16, 11, 4831-4839; Conway et Mc Laughlin (1991) Bioconjugate Chem., 2, 452-457; Letsinger et al. (1989) Proc. Natl. Acad. Sci. USA, 86,6553-6556.

De même, si nécessaire, ledit composé (haptène ou analogue) peut être modifié pour l'introduction d'une fonction réactive de façon connue en soi, en toute position appropriée sur ledit composé. Des méthodes d'introduction de fonctions réactives sont données notamment dans "Preparation of antigenic steroid-protein conjugate", F Kohen et al., dans Steroid immunoassay, Proceedings of the fifth tenovus workshop, Cardiff, Avril 1974, ed. EHD Cameron, SH. Hillier, K. Griffiths, comme par exemple l'introduction d'une fonction hémisuccinate en position 6,11, 20 ou 21, d'une fonction chloroformiate en position 11 ou d'une fonction carboxyméthyle en position 6, dans le cas de la progestérone.

L'anticorps capable de reconnaître le fragment d'acide nucléique du conjugué peut être, par exemple, un anticorps anti-fragment d'acide nucléique, et en particulier un anticorps monoclonal, tel que décrit par P. Cros et al. (Nucleic Acids Research, 22 (15), 2951-2957, 1994).

Ledit anticorps peut être lié à un traceur, en particulier par covalence.

Le traceur peut notamment être choisi parmi :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les groupements à densité électronique détectable par microscopie électronique ou par leurs propriétés électriques comme la conductivité, l'ampérométrie, la voltamétrie, les mesures d'impédance.
- les groupements détectables par des méthodes optiques comme la diffraction, la résonance plasmon de surface, la variation d'angle de contact ou des méthodes physiques comme la spectroscopie de force atomique, l'effet tunnel.

Dans un autre mode de réalisation de l'invention, l'anticorps anti-fragment d'acide nucléique n'est lié à aucun traceur, et dans ce cas, on le détecte par exemple à l'aide d'un anti-anticorps, comme indiqué ci-dessus, ledit anti-anticorps étant lui-même lié à un traceur.

Les réactions mises en jeu dans le procédé de l'invention (formation de complexes avec les anticorps, éventuellement fixation des agents marqueurs) sont effectuées dans les conditions usuelles, bien connues pour ce type de réactions, en présence d'une phase liquide (généralement une solution aqueuse tamponnée) permettant la mise en contact des réactifs et leur réaction par formation de complexes de type antigène-anticorps, par hybridation de fragments nucléiques complémentaires etc.

Selon le procédé mis en oeuvre et l'haptène à doser, un ajustement des conditions expérimentales, et notamment la détermination de la quantité d'anticorps mis en contact avec l'échantillon et de la quantité du réactif conjugué pourra être effectué préalablement, selon des méthodes bien connues de l'homme du métier, pour assurer une meilleure sensibilité et une meilleure reproductibilité. Pour cela, on peut réaliser au préalable une courbe de titrage qui permettra de déterminer la dilution de l'anticorps qui sera utilisé dans les dosages. Cette dilution correspond à une valeur de densité optique se situant dans la partie linéaire de la courbe, par exemple une valeur de densité optique comprise entre 50 % et 75 % de la valeur correspondant au seuil de saturation de la lecture. D'autres précisions sont données dans la partie expérimentale ci-après.

L'évaluation de la quantité de conjugué fixé sur les anticorps anti-haptène (ou de conjugué libre, non fixé) dans le procédé de dosage de l'invention, peut être réalisée par une méthode de détection appropriée, de façon connue en soi.

La réaction de détection peut faire intervenir par exemple :
- une première étape de fixation d'un anticorps ayant une affinité spécifique pour le fragment d'acide nucléique dudit conjugué. Cet anticorps peut être révélé à l'aide d'un agent traceur (par exemple, il est lié à l'agent traceur). Ou bien il peut être révélé, de façon connue en soi, à l'aide d'un autre anticorps capable de reconnaître ledit anticorps anti-(fragment d'acide nucléique), ledit autre anticorps étant lui-même lié à un agent traceur ;
- une seconde étape, dite de révélation, dont la nature est choisie en fonction du type de traceur utilisé pour marquer ledit agent de détection. Ce traceur et son mode de révélation sont notamment choisis parmi ceux décrits précédemment.

Il est bien entendu évident que lesdites première et seconde étapes peuvent être réalisées séparément ou simultanément, de façon connue en soi.

Par ailleurs, dans le procédé de l'invention, il est généralement utile de réaliser une étape de séparation du conjugué (et éventuellement de l'haptène) resté libre et du conjugué (et éventuellement de l'haptène) fixé sur les anticorps anti-haptène, afin d'évaluer soit la quantité de conjugué libre, soit la quantité de conjugué fixé sur les anticorps.

De nombreuses techniques, bien connues, sont utilisables pour cette séparation, et notamment:
- on peut effectuer une immunoprécipitation par un deuxième anticorps dirigés contre l'anticorps anti-haptène, ce deuxième anticorps étant apporté soit en phase liquide, soit sur phase solide, par exemple en étant fixé à des particules inertes,
- on peut utiliser des anticorps spécifiques de l'haptène à doser qui ont été préalablement fixés sur un support solide, et alors un simple lavage élimine le conjugué libre.

La fixation sur la phase solide de l'anticorps peut être réalisée par des moyens variés, soit directement par adsorption passive ou par couplage covalent, soit indirectement par l'intermédiaire d'un deuxième anticorps ou par l'intermédiaire du couple streptavidine-biotine ou analogues. Par exemple, la streptavidine sera fixée sur la phase solide et la biotine sera couplée aux anticorps spécifiques de l'haptène.

Le terme "support solide" tel qu'utilisé ici inclut tous les matériaux sur lesquels peut être immobilisée une molécule biologique pour une utilisation dans des tests diagnostiques et dans des processus de séparation. Des matériaux naturels ou de synthèse, modifiés ou non chimiquement peuvent être utilisés comme support solide, notamment les polysaccharides tels que les matériaux à base de cellulose, par exemple du papier, des dérivés de cellulose tels que l'acétate de cellulose et la nitrocellulose, du dextran; des polymères tels que polychlorures de vinyle, polyéthylènes, polystyrènes, polyacrylates, polyamides, ou des copolymères à base de monomères du type styrène, esters d'acides carboxyliques insaturés, chlorure de vinylidène, diènes ou composés présentant des fonctions nitrile (comme l'acrylonitrile); des copolymères chlorure de vinyle/propylène, chlorure de vinyle/acétate de vinyle ; des fibres naturelles telles que le coton et des fibres synthétiques telles que le nylon ; des matériaux inorganiques tels que la silice, le quartz des verres, des céramiques ; des latex, c'est-à-dire des dispersions aqueuses colloïdales d'un polymère quelconque insoluble dans l'eau; des particules magnétiques; des dérivés métalliques, etc.

Le support solide peut être notamment sous la forme d'une plaque de microtitration, d'une feuille, d'un cône, d'un tube, de billes, de particules ou analogues.

Dans les dessins annexés :

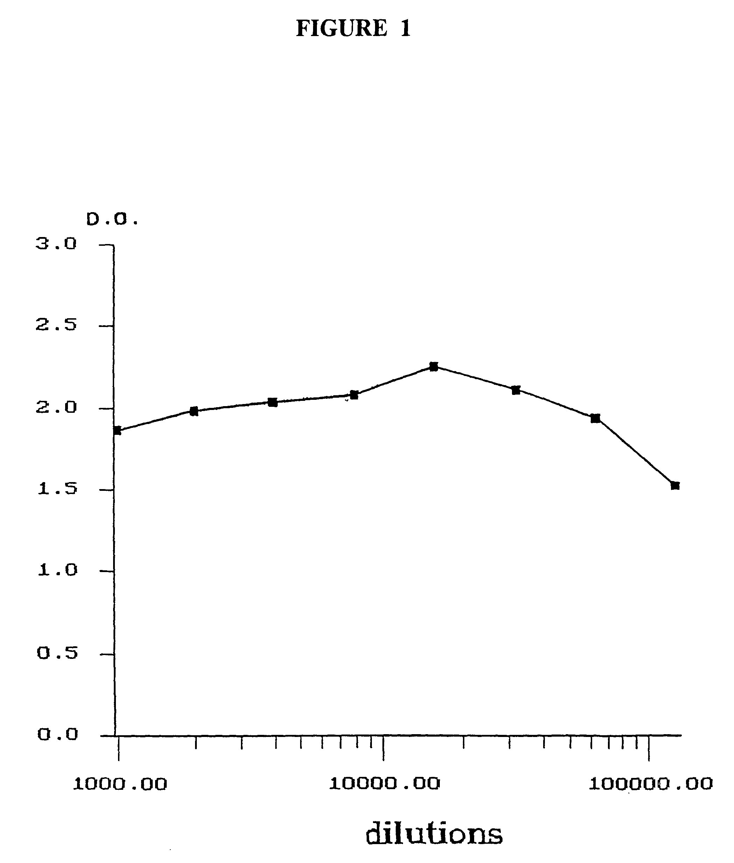

La figure 1 représente la courbe de titrage de l'anticorps monoclonal anti-estradiol obtenue dans l'exemple 2(a) ci-après, avec un conjugué estradiol phosphatase alcaline. Chaque point représente la moyenne de deux valeurs expérimentales moins la valeur de la mesure pour une fixation non spécifique.

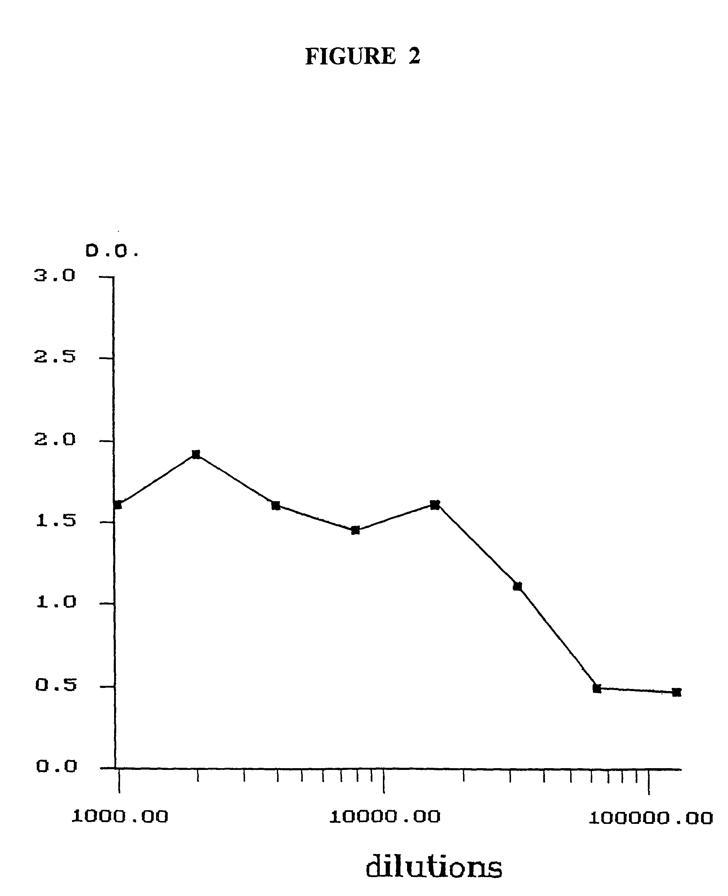

La figure 2 représente la courbe de titrage de l'anticorps monoclonal anti-estradiol obtenue dans l'exemple 2(b) ci-après, avec un conjugué estradiol-oligonucléotide, (estradiol-oligonucléotide 196 obtenu à l'exemple 1). Chaque point représente la moyenne de deux valeurs expérimentales moins la valeur de la mesure pour une fixation non spécifique.

Les exemples suivants illustrent l'invention.

### EXEMPLE 1 : Obtention de conjugués oligonucléotide-stéroïde :

Les oligonucléotides sont synthétisés sur un appareil automatique 394 de la société APPLIED BIOSYSTEMS en utilisant la chimie des phosphoramidites selon le protocole du constructeur. Afin de permettre le couplage d'un oligonucléotide sur un haptène en une position bien déterminée, des fonctions réactives sont introduites sur les oligonucléotides par l'intermédiaire de ligands compatibles avec la synthèse automatique.

Le ligand phosphoramidite (aminolink2, référence 400808 de la société Applied Biosystems) est ajouté à l'extrémité 5' de l'oligonucléotide selon le protocole standard de la synthèse automatique. Ainsi, la fonction réactive, utilisée pour le greffage sur un haptène, est localisée à l'extrémité 5' de l'oligonucléotide.

Après déprotection une nuit à 55°C dans une solution à 33 % de NH₄OH et précipitation en présence d'éthanol à -20°C, les oligonucléotides sont séchés sous vide et repris dans 1 ml d'H₂O.

Les oligonucléotides modifiés sont ensuite purifiés par chromatographie liquide haute pression (CLHP) en phase inverse sur une colonne Brownlee RP18 (10 mm - 25 cm).
- Conditions :: débit 4,6 ml/min
Gradient de 10 % à 35 % de tampon B en 30 min.
Gradient de 35 % à 100 % de tampon B en 3 min
avec
- Tampon B :: 50 % Tampon A + 50 % CH₃CN.
et
- Tampon A :: acétate de triéthylammonium (TEAA) 0,1M; pH = 7,00

Les oligonucléotides modifiés par le bras aminolink2 et utilisés dans la présente invention sont décrits dans le tableau 1.

**TABLEAU 1**

| **Numéro usuel de l'oligonucléotide** | **SEQ ID N°** | **Nucléotide** | **Tr** |
|---|---|---|---|
| 57 | 1 | bêta | 19,54 |
| 196 | 2 | alpha | 23,21 |

Les nucléotides bêta sont les nucléotides naturels, c'est-à-dire que la liaison glycosidique est sous la forme anomérique bêta, contrairement aux nucléotides alpha, dans lesquels la liaison glycosidique est sous la forme anomérique alpha. Les oligonucléotides contenant les nucléotides alpha ont été préparés selon la publication de F. MORVAN et collaborateurs, Nucleic Acid Research, 16(3), pages 833-847, 1988.

Tr représente le temps de rétention en minutes de l'oligonucléotide dans les conditions suivantes :
Colonne Brownlee RP18 (4,6 mm - 25 cm).
- Conditions :: débit 1 ml/min
Gradient de 10 % à 35 % de tampon B en 30 min.
Gradient de 35 % à 100 % de tampon B en 3 min.

### Couplage de l'oligonucléotide modifié synthétisé sur un haptène :

Les dérivés d'haptènes utilisés sont décrits dans le tableau 2.

**TABLEAU 2**

| **Haptène à doser** | **Dérivé d'haptène utilisé pour le couplage** | **Fournisseur** |
|---|---|---|
| ESTRADIOL | ESTRADIOL-6- CARBOXYMETHOXIME-N- HYDROXYSUCCINIMIDE ESTER | BOEHRINGER MANNHEIM |
| TESTOSTERONE | TESTOSTERONE-19- HEMISUCCINATE-N- HYDROXYSUCCINIMIDE ESTER | BIO MERIEUX |

Le dérivé de testostérone, fourni par BIO MERIEUX, est préparé selon la méthode décrite dans J. Steroid Biochem., 23(6a), p 981-989, 1985 par A. WHITE et al.

L'oligonucléotide (50 nmoles) est séché à l'évaporateur rotatif et repris dans 20 µl de tampon borate de sodium 0,1 M, pH 9,3. On ajoute 300 µl d'une solution de dérivé d'haptène (5 mg/ml dans le DMF) goutte à goutte et le mélange obtenu est placé sous agitation pendant 3 heures à 50°C. Après centrifugation, le surnageant est séparé et le culot de centrifugation est repris dans 100 µl d'eau. Le surnageant réservé est alors ajouté et on ajoute au mélange obtenu 250 µl d'acétate d'ammonium 3M pH 5,2, et 5 ml d'éthanol absolu froid (-20°C). Après incubation 30 min à -80°C et centrifugation, le culot est repris dans 500 µl d'eau.

Le conjugué oligonucléotide-haptène obtenu est purifié par CLHP dans les conditions suivantes :
Colonne BECKMAN ODS (10 mm - 25 cm).
- Conditions :: débit 4,6 ml/min
Gradient de 20 % à 30 % de tampon B en 10 min.
Gradient de 30 % à 70 % de tampon B en 20 min.

Le conjugué recueilli est séché à l'évaporateur rotatif puis repris dans 500 µl d'eau.

De façon analogue, on a préparé différents conjugués oligonucléotide-haptène. La nature des conjugués obtenus est résumée dans le tableau 3.

**TABLEAU 3**

| **Conjugué oligonucléotide- haptène** | **Oligonucléotide** | **Haptène** | **Tr** |
|---|---|---|---|
| A | 57 | ESTRADIOL | 16,18 |
| B | 196 | ESTRADIOL | 15,65 |
| C | 57 | TESTOSTERONE | 27,96^{(a)} |

Dans le tableau 3, Tr est le temps de rétention du produit purifié dans les conditions suivantes:
Colonne Brownlee RP 300 (4.6 mm x 100 mm)
- Conditions :: débit 1,0 ml/mn
Gradient de 20 % à 30 % de tampon B en 10 min.
Gradient de 30 % à 70 % de tampon B en 20 min.
(a) pour le conjugué C le gradient est de 10 à 40% de B en 30 min.

### EXEMPLE 2 : Dosage de l'estradiol par compétition

### (a) Selon l'art antérieur

(i) Un anticorps monoclonal anti-estradiol obtenu par la technique décrite par G. Köhler et C. Milstein (Nature 256. 495-497 (1975) est obtenu après immunisation de souris BALB-c avec un conjugué estradiol-albumine bovine (ce conjugué étant obtenu par réaction du dérivé d'estradiol du tableau 2 avec l'albumine), est utilisé à une concentration déterminée préalablement par établissement d'une courbe de titrage.
   La courbe de titrage est obtenue comme suit : dans les puits d'une plaque de microtitration en polystyrène Maxisorb NUNC (commercialisée par la société Polylabo Paul Block sous la référence 4-39454), on dépose, à raison de 100 µl par puits, une solution à 5 µg/ml d'anticorps de chèvre anti-(IgG (H + L) de souris) (commercialisé par la société Jackson Immuno Research Laboratories sous la référence 115-005-062) dans un tampon bicarbonate (NaHCO₃ 0,05 M; pH 9,6). Cette plaque est mise en incubation une nuit à 22°C ou une heure à 37°C. La plaque est lavée trois fois par 300 µl de tampon PBS-Tween 0,05 % (NaCl 0,15 M; phosphate de sodium 0,05 M; pH 7,0; Tween 20 à une concentration finale de 0,05 % (Tween : nom commercial)). Les sites dans la plaque non occupés par l'anticorps sont saturés par l'addition de 100 µl d'une solution de PBS-lait à 1 % en concentration finale (NaCl 0,15 M; phosphate de sodium 0,05 M; pH 7,0; lait écrémé Régilait (nom commercial) 1 %). On fait incuber pendant une heure à 37°C, puis on rince 3 fois par 300 µl de tampon PBS-Tween 0,05 %. Dans chaque puits de la plaque, 100 µl de dilutions successives de l'anticorps anti-estradiol en tampon PBS sont ajoutés. Après incubation pendant 1 heure à 37°C, la plaque est rincée trois fois avec du tampon PBS-Tween 0,05 %. On ajoute dans chaque puits 50 µl de sérum humain masculin commercialisé par Bio Mérieux réf. 66581) et 50 µl de conjugué estradiol-phosphatase alcaline (l'estradiol est conjugué à la phosphatase alcaline par formation d'un ester de N-hydroxysuccinimide et couplage par le dicyclohexylcarbodiimide comme décrit par Mattox et al. J. Steroid Biochem., 10, p. 167-172, 1979). On laisse incuber pendant 1 heure à 37°C.
   La plaque est rincée trois fois avec du tampon PBS-Tween 0,05 %, puis une fois avec de l'eau distillée. On ajoute dans chaque puits 100 µl de substrat PNPP (paranitrophényl-phosphate commercialisé par la société Sigma sous la référence 104-0) à la concentration de 2mg/ml dans du tampon diéthanolamine-HCl (diéthanolamine 1,29 M; MgSO4 0,56 mm, NaNO₃ 38 mm; HCl 0,012 N; pH 9,8). Après une incubation de 30 minutes à 37°C, la réaction enzymatique est bloquée par addition de 100 µl de NaOH 1 N . La lecture est effectuée à 405 nm sur un lecteur Biowhittaker Microplate Reader 2001. La courbe de titrage obtenue est représentée à la figure 1.
   Cette courbe de titrage permet de déterminer la dilution de l'anticorps anti-estradiol qui sera utilisée ultérieurement et qui correspond par exemple à une DO de 1,5-2 à 405 nm.
(ii) Le dosage de la molécule d'estradiol dans des conditions de compétition est effectué selon un protocole analogue, mais dans ce cas, l'étape d'addition du conjugué estradiol-phosphatase alcaline est remplacée par l'addition de 50 µl d'une solution choisie parmi des solutions de concentration croissante d'estradiol, diluées dans du sérum humain masculin dépourvu d'haptènes (BIOMERIEUX Réf. 66581), et de 50 µl du conjugué estradiol-phosphatase alcaline préparé comme décrit ci-dessus. L'ensemble est mis en incubation pendant 1 heure à 37°C.

Les mesures sont effectuées après une étape de révélation en présence du substrat de l'enzyme, comme décrit précédemment. Les résultats sont présentés dans le tableau 4 :

**TABLEAU 4**

| **Concentration d'estradiol pg/ml** | **D.O. 405 nm (*)** |
|---|---|
| 0 | 1,755 |
| 10 | 1,749 |
| 100 | 1,646 |
| 1000 | 1,194 |
| 10000 | 0,289 |
| 100000 | 0,078 |

| | |
|---|---|
| (*) chaque résultat représente la DO moyenne de deux expériences moins la valeur de la fixation non spécifique (lecture 405 nm). | |

Les résultats de la gamme d'étalonnage présentés dans le tableau 4 montrent que l'on obtient une inhibition de la fixation du conjugué estradiol-phosphatase alcaline lorsque la concentration en estradiol augmente. La valeur représentant 50 % d'inhibition de la fixation du conjugué estradiol phosphatase alcaline correspond à une concentration de 2200 pg/ml d'estradiol.

### (b) Utilisation d'un conjugué oligonucléotide-estradiol révélé par l'intermédiaire d'un anticorps marqué anti-oligonucléotide

Sauf indications contraires, les appareils et réactifs utilisés sont les mêmes que précédemment.
(i) La courbe de titrage est obtenue comme suit : dans les puits d'une plaque de microtitration, on dépose à raison de 100 µl par puits, une solution à 1 µg/ml d'un anticorps de chèvre anti-(IgG (H + L) de souris) dans un tampon bicarbonate (NaHCO₃ 0,05 M; pH 9,6). On laisse incuber une nuit à 22°C ou une heure à une température de 37°C. La plaque est lavée trois fois par 300 µl de tampon PBS-Tween 0,05 % (NaCl 0,15 M; phosphate de sodium 0,05 M ; pH 7,0; Tween 20 à une concentration finale de 0,05 % (Tween : nom commercial)). Les sites de la plaque non occupés par l'anticorps sont saturés par l'addition de 100 µl d'une solution de PBS-lait à 1 % en concentration finale (NaCl 0,15 M; phosphate de sodium 0,05 M; pH 7,0; lait écrémé Régilait (nom commercial) 1 %). Après incubation pendant une heure à 37°C, on rince 3 fois par 300 µl de tampon PBS-Tween 0,05 %. Dans chaque puits de la plaque on ajoute 100 µl de dilutions successives différentes de la solution d'anticorps anti-estradiol en tampon PBS et on laisse incuber pendant 1 heure à 37°C. La plaque est rincée trois fois avec du tampon PBS-Tween 0,05 %, puis les sites non occupés par l'anticorps anti-estradiol sont saturés par l'addition de 100 µl d'une solution de PBS-(NaCl 0,15 M; phosphate de sodium 0,05 M; pH 7,0) contenant 10 % de liquide d'ascite de souris produit à partir de la lignée de cellules Sarcome T-180 (commercialisé par BIO MERIEUX sous la référence 30202). On laisse incuber pendant une heure à 37°C puis on rince 3 fois par 300 µl de tampon PBS-Tween 0,05 %. Dans chaque puits de la plaque 50 µl de sérum humain d'homme sont ajoutés et sont mélangés à 50 µl de conjugué B (voir exemple 1) en solution à 0,72 nM dans le PBS. On fait incuber 1 heure à 37°C puis on lave trois fois par 300 µl de tampon PBS-Tween 0,05 %. Par ailleurs, l'anticorps monoclonal anti-oligonucléotides décrit par P. Cros et al., article cité ci-dessus, a été purifié à partir de liquides d'ascites par chromatographie de type échange d'ions ABx (Backer 726900), puis conjugué à la phosphatase alcaline (Boehringer) selon la technique de S. Avrameas (Immunochem., 6, p. 43, 1969). On prépare une solution de cet anticorps diluée au 1/400 (concentration totale en protéine 1 mg/ml) en tampon PBS - sérum humain masculin 1 % - sérum normal de chèvre 1 % - liquide d'ascite de souris (de la lignée sarcome T-180) 10 %. 100 µl de cette solution d'anticorps sont ajoutés dans les puits de la plaque. Après une incubation pendant 1 heure à 37 °C et trois rinçages avec du tampon PBS-Tween 0,05 % puis un rinçage avec de l'eau distillée, 100 µl de substrat PNPP à la concentration de 2 mg/ml dans du tampon diéthanolamine-HCl (diéthanolamine 1,29 M ; MgSO₄ 0,56 mm, NaNO₃ 38 mm; HCl 0,012 N; pH 9,8) sont ajoutés dans les puits de la plaque. On laisse incuber 30 minutes à 37°C puis la réaction est bloquée par addition de 100 µl de NaOH 1 N. La lecture des plaques est effectuée à 405 nm sur un lecteur Biowhittaker Microplate Reader 2001. La courbe de titrage obtenue est représentée à la figure 2. Cette courbe de titrage permet de déterminer la dilution de l'anticorps anti-estradiol qui sera utilisée et qui correspond par exemple à une DO de 1-1,5 à 405 nm.
(ii) Le dosage de la molécule d'estradiol dans des conditions de compétition est ensuite effectué selon le même protocole qu'à l'étape(i) ci-dessus, mais dans ce cas 50 µl d'une solution choisie parmi des solutions de concentration croissante d'estradiol, diluées en sérum humain dépourvu d'haptènes (Bio Mérieux réf. 66581) sont ajoutés aux 50 µl de la solution 0,72 nM de conjugué B, puis on laisse en incubation pendant 1 heure à 37°C .

La suite de l'expérimentation se déroule comme décrit précédemment. Les résultats sont présentés dans le tableau 5 :

**TABLEAU 5**

| **Concentration d'estradiol pg/ml** | **D.O. 405 nm (*)** |
|---|---|
| 0 | 1,551 |
| 10 | 1,277 |
| 100 | 1,210 |
| 1000 | 0,731 |
| 10000 | 0,190 |
| 100000 | 0,130 |

| | |
|---|---|
| (*) chaque résultat représente la DO moyenne de deux valeurs moins la valeur de la fixation non spécifique (lecture 405 nm). | |

Ces résultats de dosage réalisé selon une technique de compétition montrent que l'on obtient une inhibition de la fixation du complexe (conjugué B/anticorps anti-oligonucléotide-phosphatase alcaline) lorsque la concentration en estradiol augmente. La valeur représentant 50 % d'inhibition de la fixation dudit complexe correspond à une concentration de 900 pg/ml d'estradiol.

De façon analogue, en utilisant un anticorps anti-testostérone, le conjugué C (voir exemple 1) et, comme marqueur, des anticorps dirigés contre l'oligonucléotide 57 (voir exemple 1), on améliore la sensibilité du dosage de la testostérone, par rapport à un dosage selon l'art antérieur effectué de façon similaire à celui décrit à l'exemple 2(a).

## Revendications

1. Procédé pour le dosage d'un haptène dans un échantillon, dans lequel : (a) on met en contact ledit échantillon avec une quantité prédéterminée d'anticorps capables de reconnaître ledit haptène et avec une quantité prédéterminée d'un réactif capable d'entrer en compétition avec ledit haptène pour la fixation sur lesdits anticorps par formation d'un complexe de type antigène-anticorps, ledit réactif étant un conjugué formé par un fragment d'acide nucléique simple brin avec un composé capable d'être reconnu par les anticorps reconnaissant l'haptène, et (b) on détermine, à l'aide d'un marqueur capable de se lier avec ledit réactif, la quantité dudit réactif fixé sur lesdits anticorps ou la quantité dudit réactif non fixé sur lesdits anticorps, ledit marqueur étant un anticorps capable de reconnaître ledit fragment d'acide nucléique.

2. Procédé selon la revendication 1, caractérisé par le fait que l'on détermine la quantité de réactif fixé ou non fixé en mesurant la quantité de marqueur lié soit au réactif fixé sur les anticorps anti-haptène, soit au réactif libre, non fixé.

3. Procédé selon l'une quelconque des revendications précédentes, caractérisé par le fait que ledit fragment d'acide nucléique contenant au plus 100 nucléotides

4. Procédé selon l'une quelconque des revendications précédentes, caractérisé par le fait que ledit fragment d'acide nucléique contient au plus 40 nucléotides.

5. Procédé selon l'une quelconque des revendications précédentes, caractérisé par le fait que l'on mélange ledit échantillon et ledit réactif avant la mise en contact avec les anticorps capables de reconnaître l'haptène.

6. Procédé selon l'une quelconque des revendications 1 à 4, caractérisé par le fait que dans un premier temps on met en contact ledit échantillon avec lesdits anticorps capables de reconnaître l'haptène et que dans un second temps on ajoute ledit réactif.

## Patentansprüche

1. Verfahren für die Dosierung eines Haptens in einer Probe, in dem:
(a) die Probe in Kontakt gebracht wird mit einer vorbestimmten Menge an Antikörpern, die das Hapten erkennen können, und mit einer vorbestimmten Menge eines Reagens, das durch Bildung eines Komplexes vom Typ Antigen/Antikörper mit dem Hapten bezüglich der Fixierung auf den Antikörpern in Konkurrenz treten kann, wobei das Reagens ein Konjugat ist, gebildet aus einem einsträngigen Nukleinsäurefragment und einer Verbindung, die von den Antikörpern, welche das Hapten erkennen, erkannt werden kann, und
(b) mit Hilfe eines Markers, der in der Lage ist, sich mit dem Reagens zu verbinden, die Menge des auf den Antikörpern fixierten Reagens oder die Menge des nicht auf den Antikörpern fixierten Reagens bestimmt wird, wobei der Marker ein Antikörper ist, der das Nukleinsäurefragment erkennen kann.

2. Verfahren nach Anspruch 1, dadurch gekennzeichnet, dass die Menge des fixierten oder nicht fixierten Reagens bestimmt wird, indem die Menge des Markers, der entweder an das auf den Antihapten-Antikörpern fixierte Reagens oder an das freie, nicht fixierte Reagens gebunden ist, gemessen wird.

3. Verfahren nach einem der vorstehenden Ansprüche, dadurch gekennzeichnet, dass das Nukleinsäurefragment höchstens 100 Nukleotide enthält.

4. Verfahren nach einem der vorstehenden Ansprüche, dadurch gekennzeichnet, dass das Nukleinsäurefragment höchstens 40 Nukleotide enthält.

5. Verfahren nach einem der vorstehenden Ansprüche, dadurch gekennzeichnet, dass die Probe und das Reagens vor dem in-Kontakt-bringen mit den Antikörpern, welche das Hapten erkennen können, gemischt werden.

6. Verfahren nach einem der vorstehenden Ansprüche 1 bis 4, dadurch gekennzeichnet, dass man zuerst die Probe mit den Antikörpern, welche das Hapten erkennen können, in Kontakt bringt und dann das Reagens zugibt.

## Claims

1. Method of assaying a hapten in a sample, in which: (a) the said sample is brought into contact with a predetermined quantity of antibodies capable of recognizing the said hapten and with a predetermined quantity of a reagent capable of competing with the said hapten for binding to the said antibodies by forming an antigen-antibody-type complex, the said reagent being a conjugate formed by a single-stranded nucleic acid fragment with a compound capable of being recognized by the antibodies recognizing the hapten, and (b) the quantity of the said reagent bound to the said antibodies or the quantity of the said reagent not bound to the said antibodies is determined with the aid of a marker capable of attaching to the said reagent, the said marker being an antibody capable of recognizing the said nucleic acid fragment.

2. Method according to Claim 1, characterized in that the quantity of bound or unbound reagent is determined by measuring the quantity of marker attached either to the reagent bound to the anti-hapten antibodies, or to the free, unbound reagent.

3. Method according to any one of the preceding claims, characterized in that the said nucleic acid fragment contains at most 100 nucleotides.

4. Method according to any one of the preceding claims, characterized in that the said nucleic acid fragment contains at most 40 nucleotides.

5. Method according to any one of the preceding claims, characterized in that the said sample and the said reagent are mixed before the bringing into contact with the antibodies capable of recognizing the hapten.

6. Method according to any one of Claims 1 to 4, characterized in that, in a first instance, the said sample is brought into contact with the said antibodies capable of recognizing the hapten and in that, in a second instance, the said reagent is added.
